Europäisches Patentamt

European Patent Office

Office européen des brevets

(19)

(11) Veröffentlichungsnummer: **0 024 327**
**B1**

(12) **EUROPÄISCHE PATENTSCHRIFT**

(45) Veröffentlichungstag der Patentschrift:
12.01.83

(21) Anmeldenummer: 80104591.5

(22) Anmeldetag: 04.08.80

(51) Int. Cl.³: **A 61 B 5/08**, G 01 P 5/10,
G 01 F 1/68

(54) Atemstrommesser mit Richtungsbestimmung.

(30) Priorität: 16.08.79 DE 2933116

(43) Veröffentlichungstag der Anmeldung:
04.03.81 Patentblatt 81/9

(45) Bekanntmachung des Hinweises auf die Patenterteilung:
12.01.83 Patentblatt 83/2

(84) Benannte Vertragsstaaten:
AT BE CH DE FR GB IT LI NL SE

(56) Entgegenhaltungen:
DE-B-2 317 431
DE-B-2 318 279
US-A-3 645 133
US-A-3 962 917
US-A-4 083 244
SOVIET INVENTIONS ILLUSTRATED, Sektion P, Q, Woche B 51, 6. Feber 1980 Derwent Publications LTD. London, P 31

(73) Patentinhaber: Rico Gesellschaft für Mikroelektronik mbH, Industriestrasse 51-53, D-8963 Waltenhofen (DE)

(72) Erfinder: Willam, Franz, Haubenschlossstrasse 17, D-8960 Kempten (DE)

(74) Vertreter: Hübner, Hans-Jürgen, Dipl.-Ing., Mozartstrasse 21, D-8960 Kempten (DE)

Atemstrommesser mit Richtungsbestimmung

Die Erfindung betrifft einen Atemstrommesser mit Richtungsbestimmung mit einem in die Atemluftleitung eingebauten Messkopf, der einen im Luftströmungsweg angeordneten Luftwiderstandskörper sowie zwei in rechtwinklig zur Luftströmung liegenden Radialebenen vor und hinter dem Luftwiderstandskörper angeordnete Temperaturfühler aufweist, von denen einer im Strömungsbeeinflussungsbereich des Luftwiderstandskörpers und mit diesem in derselben Axialebene liegt und die beide mittels getrennter elektrischer oder elektronischer Temperatur-Konstanthaltungskreise auf einer konstanten, gegenüber der Atemlufttemperatur erhöhten Arbeitstemperatur gehalten werden, mit einem die elektrische Energie zur Kompensation der bei Atmung oder Beatmung erfolgenden Abkühlung jeweils eines Fühlers als Wert für den Luftdurchsatz messenden Messgerät und mit Differenzbildung der Energiezufuhrwerte zur Bestimmung der Strömungsrichtung.

Solche Atemstrommesser mit Richtungsbestimmung sind bekannt (DE-B2-2 317 431, US-A-4 083 244). Die beiden Fühler liegen in derselben Strömungsebene, und zwischen ihnen in geringem Abstand befindet sich der Luftwiderstandskörper. Die beiden Fühler werden vom Luftstrom unterschiedlich stark gekühlt. Die zugeführte Energie ist beim einen Fühler geringer als beim anderen. Das Differenzsignal der Energiezufuhrwerte beider Fühler ist bei Strömung in einer Richtung positiv und in der entgegengesetzten Richtung negativ. Auf diese Weise ist eine einwandfreie Richtungsbestimmung des Atemstromes möglich.

Bei beiden bekannten Vorschlägen wird die Energiezufuhr des stromaufseitigen Temperaturfühlers als Mass für den Luftdurchsatz benutzt. Vor jedem Luftwiderstandskörper wird jedoch die Strömung ebenfalls mehr oder weniger beeinflusst und zwar in Abhängigkeit vom Abstand des Fühlers vom Widerstandskörper und von der Strömungsgeschwindigkeit. Der stromaufseitige Fühler kann also in einer Zone liegen, in der die Strömungsgeschwindigkeit Null ist. In den meisten Fällen ist sie jedenfalls kleiner als die Geschwindigkeit der ungestörten Strömung. Bei der Luftdurchsatzbestimmung wird somit ein zu kleiner Wert ermittelt. Der Fehler ist nicht kompensierbar, da er von der Strömungsgeschwindigkeit abhängig ist.

Aufgabe der Erfindung ist es, den bekannten Atemstrommesser ohne baulichen Mehraufwand dahingehend zu verbessern, dass eine exakte Bestimmung des Durchsatzes der Atemluft ermöglicht wird.

Diese Aufgabe wird erfindungsgemäss dadurch gelöst, dass der andere Fühler von der den ersten Fühler und den Luftwiderstandskörper enthaltenden Axialebene einen Radialabstand hat, der ausreicht, um ihn aus dem Bereich der Strömungsbeeinflussung durch den Widerstandsköper herauszuhalten, wobei dieser Fühler unabhängig von der Strömungsrichtung zur Messung des Luftdurchsatzes dient.

Zwar ist schon ein Atemstrommessr bekannt (US-A-3645133), bei dem zwei Fühler in unterschiedlichem Radialabstand verwendet werden, allerdings fehlt ein Luftwiderstandskörper. Ein Fühler dient der Durchsatzbestimmung und der andere radialversetzte Fühler dient nur der Temperaturkompensation bei unterschiedlichen Umgebungstemperaturen. Eine Richtungsbestimmung durch Bildung eines Differenzsignals ist mit diesem Atemstrommesser nicht möglich, weil beide Fühler in einer ungestörten Strömung liegen und in beiden Strömungsrichtungen ein Differenzsignal immer den Wert Null haben würde.

Dank der Erfindung dass einer der beiden Fühler aus der Strömungsachse des Luftwiderstandskörpers radial versetzt wird, kann dieser Fühler vom Luftwiderstandskörper strömungsmässig nicht mehr beeinflusst werden, ganz gleich, in welcher Richtung die Strömung verläuft. Dieser Fühler misst daher den Luftdurchsatz fehlerfrei. Die ihm zugeführte Energie ist bei Strömung in beiden Richtungen grösser als die des anderen Fühlers. Die Differenzsignale sind immer positiv, jedoch je nach Strömungsrichtung unterschiedlich gross. Die Richtungsbestimmung ist somit ebenfalls fehlerfrei möglich.

Weitere Ausgestaltungen der Erfindung bilden die Gegenstände der Unteransprüche.

Es zeigt:

Figur 1 einen vertikalen Längsschnitt durch den neuen Atemstrommesser

Figur 2 eine Stirnansicht des Atemstrommessers, und

Figur 3 eine Längsschnittansicht längs der Linie 3-3 der Figur 1

Ein Messkopf 10 besteht aus einem rohrförmigen Gehäuse 12, in dem ein Störmungskanal gebildet wird, der zwei stirnseitige Anschlussstutzen 14, 16, einen Verengungsabschnitt 18, einen Erweiterungsabschnitt 20, sowie einen dazwischen liegenden zylindrischen Mittelabschnitt 22 aufweist. Ein Sieb 26 hält grobe Sektretteile und sonstige Fremdkörper zurück.

Der Mittelabschnitt 22 hat eine Bohrung, in welche ein Isolierkörper 28 eingesetzt ist, der von zwei langen Stiften 31, 33 durchsetzt ist, deren innere Enden durch einen Platindraht 35 miteinander verbunden sind und deren äussere Enden Kontaktstifte für eine nicht dargestellte Steckkupplung bilden. Der Isolierkörper 28 weist weiterhin zwei kurze Stifte 36, 38 mit einem Platindraht 40 und aussen einen Orientierungsstift 44 auf.

Die beiden Platindrähte 35, 40 liegen in Radialebenen zwischen denen in Axialabständen ein parallel zu ihnen verlaufender Bolzen oder Steg 42 im Gehäusemittelabschnitt 22 gehalten ist. Dabei liegen der Platindraht 40 und der Bolzen 42 in derselben mittleren Axialebene des Strömungskanals während der andere Platindraht 35 gegenüber dieser Axialebene radial versetzt ist.

Die beiden Platindrähte 35, 40 stellen Temperaturmessfühler dar, die an eine elektronische Messschaltungsanordnung angeschlossen sind, welche für jeden Fühler einen elektronischen Regelkreis umfasst, der sie jeweils auf einer hohen Temperatur von etwa 800 °C konstant hält. Strömt Atemluft durch den Strömungskanal 24, werden beide Fühler 35, 40 gekühlt, der Fühler 35 jedoch stärker als der Fühler 40, weil er in einer vom Bolzen 42 unbeeinflussten Strömung liegt. Die zur Temperaturkonstanthaltung notwendigen Energiemengen beider Fühler werden mittels bekannter elekronischer Regelkreise getrennt gemessen, linearisiert und verstärkt. Der Wert des Fühlers 35 dient, mit einem Eichfaktor multipliziert, direkt zur Bestimmung des momentanen Luftdurchsatzes.

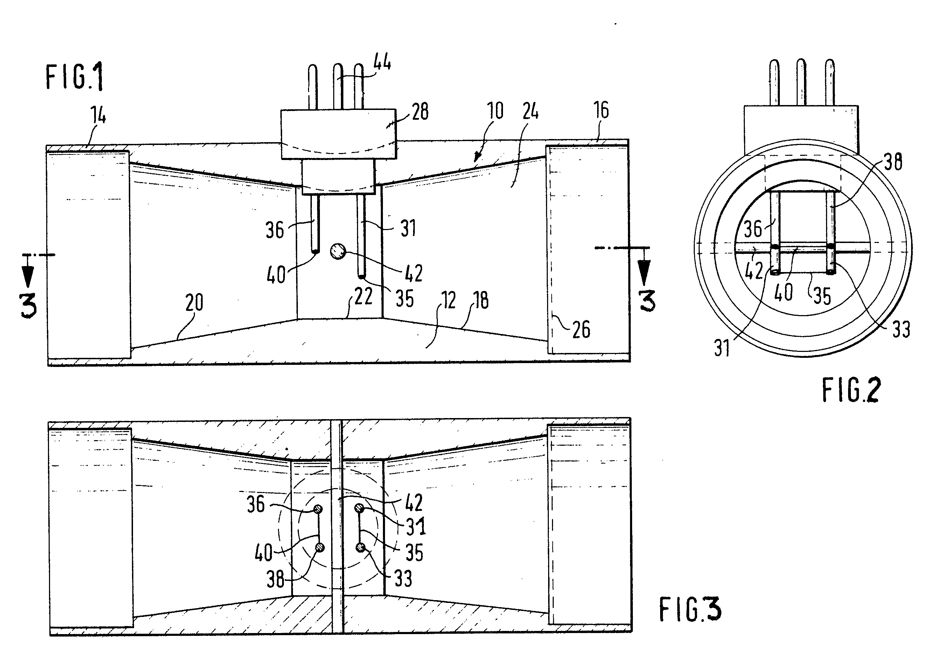

Zur Richtungsbestimmung (Beatmen, Ausatmen) wird aus den momentanen Messwerten beider Fühler 35, 40 ein Differenzsignal erzeugt, das bei Strömung von rechts nach links (Figur 1) einen positiven grossen Wert und in entgegengesetzter Strömungsrichtung einen positiven kleinen Wert hat, weil im ersten Fall der Fühler 40 im Windschatten des Bolzens 42 liegt und sich erheblich weniger abkühlt als Fühler 35, während im zweiten Fall der jetzt stromauf des Bolzens 42 liegende Fühler 40 stärker gekühlt wird aber wegen eines gewissen Strömungsstaus doch etwas geringer als der in ungestörter Strömung liegende Fühler 35.

Das neue Gerät ermittelt Strömungsrichtung, Zugvolumen, Minutenvolumen der Atemfrequenz, Exspirations- und Inspirationszeiten, sowie das Atem-Zeitverhältnis.

**Patentansprüche**

1. Atemstrommesser mit Richtungsbestimmung mit einem in die Atemluftleitung eingebauten Messkopf (10), der einen im Luftströmungsweg angeordneten Luftwiderstandskörper (42) sowie zwei in rechtwinklig zur Luftströmung liegenden Radialebenen vor und hinter dem Luftwiderstandskörper (42) angeordnete Temperaturfühler (35, 40) aufweist, von denen einer (40) im Strömungsbeeinflussungsbereich des Luftwiderstandskörpers (42) und mit diesem in derselben Axialebene liegt und die beide mittels getrennter elektrischer oder elektronischer Temperatur-Konstanthaltungskreise auf einer konstanten gegenüber der Atemlufttemperatur erhöhten Arbeitstemperatur gehalten werden, mit einem die elektrische Energie zur Kompensation der bei Atmung oder Beatmung erfolgenden Abkühlung jeweils eines Fühlers als Wert für den Luftdurchsatz messenden Messgerät und mit Differenzbildung der Energie-Zufuhrwerte zur Bestimmung der Strömungsrichtung, dadurch gekennzeichnet, dass der andere Fühler (35) von der den ersten Fühler (40) und den Luftwiderstandskörper (42) enthaltenden Axialebene einen Radialabstand hat, der ausreicht, um ihn aus dem Bereich der Strömungsbeeinflussung durch den Widerstandskörper (42) herauszuhalten, wobei dieser Fühler (35) unabhängig von der Strömungsrichtung zur Messung des Luftdurchsatzes dient.

2. Atemstrommesser nach Anspruch 1, dadurch gekennzeichnet, dass die Fühler (35, 40) je aus dünnen Platindrähten bestehen.

3. Atemstrommesser nach Anspruch 2, dadurch gekennzeichnet, dass die Platindrähte (35, 40) je zwischen zwei Stiften (31, 33 bzw. 36, 38) angeordnet sind und sich parallel zueinander und quer zur Luftströmungsrichtung erstrecken.

4. Atemstrommesser nach einem der Ansprüche 1 bis 3, dadurch gekennzeichnet, dass der Luftwiderstandskörper (42) aus einem sich quer zur Luftströmungsrichtung durch den Luftströmungskanal (24) erstreckenden Bolzen oder Steg besteht.

5. Atemstrommesser nach einem der Ansprüche 1 bis 4, dadurch gekennzeichnet, dass der Luftströmungskanal (24) sich aus einem Verengungsabschnitt (18), einem Mittelabschnitt (22) mit gleichbleibendem Querschnitt und einem Erweiterungsabschnitt (20) zusammensetzt und dass die Fühler (35, 40) und der Luftwiderstandskörper (42) dem Mittelabschnitt (22) zugeordnet sind.

**Revendications**

1. Appareil de mesure du courant respiratoire avec détermination de son sens, comprenant une tête de mesure (10) montée dans la conduite d'air respiratoire, où se trouve un obstacle aérodynamique (42) disposé sur le trajet d'élement de l'air, ainsi que deux capteurs de température (35, 40) disposés dans des plans radiaux placés perpendiculairement à l'écoulement de l'air, en amont et en aval de l'obstacle aérodynamique (42), dont l'un (40) se trouve dans la région d'influence de l'bostacle aérodynamique (42) sur l'écoulement et dans le même plan axial que ce dernier, et qui sont maintenus tous deux, au moyen de circuits électriques ou électroniques séparés de maintien, à une température constante, ladite température de travail étant, élevée par rapport à la température de l'air de respiration, ledit appareil de mesure mesurant comme valeur pour le débit d'air, l'énergie électrique nécessaire pour compenser le refroidissement de chaque capteur qui se reproduit lors de l'expiration ou de l'inspiration et pour la détermination du sens de l'écoulement la différence des valeurs d'énergie fournie, caractérisé en ce que l'autre capteur (35) se trouve à une distance radiale du plan axial contenant le premier capteur (40) et l'obstacle aérodynamique (42) qui suffit pour le maintenir en dehors de la région d'influence sur l'écoulement de l'obstacle aérodynamique (42), ce capteur servant à mesurer le débit) d'air indépendamment du sens de l'écoulement.

2. Appareil de mesure du courant respiratoire suivant la revendication 1, caractérisé en ce que les capteurs (35, 40) sont constitués chacun par des fils de platine fins.

3. Appareil de mesure du courant respiratoire suivant la revendication 2, caractérisé en ce que les fils de platine (35, 40) sont disposés chacun entre deux broches (31, 33 ou 36, 38 respectivement) et s'étendent parallèlement entre eux et transversalement à la direction de l'écoulement de l'air.

4. Appareil de mesure du courant respiratoire suivant l'une des revendications 1 à 3, caractérisé en ce que l'obstacle aérodynamique (42) est constitué par une tige ou une traverse qui s'étend dans le canal d'écoulement de l'air (24) transversalement à la direction d'écoulement de l'air.

5. Appareil de mesure du courant respiratoire suivant l'une des revendications 1 à 4, caractérisé en ce que le canal d'écoulement de l'air (24) se compose d'un segment convergent (18), d'un segment central (22) à section constante et d'un segment divergent (20) et en ce que les capteurs (35, 40) et l'obstacle aérodynamique (42) sont combinés au segment central (22).

**Claims**

1. Spirometer with direction determination comprising a measuring head (10) installed in the respiration conduit, the measuring head provided with an air resistance body (42) and a pair of temperature sensors (35, 40), the air resistance body (42) arranged in the air flow path and the temperature sensors (35, 40) arranged in a pair of radial planes which form right angles with the air flow, one of the sensors arranged upstream and the other one downstream of the air resistance body (42), one (40) of the sensors (35, 40) situated in the flow influence area of the air resistance body (42) and with it in the same axial plane, both of the temperature sensors (35, 40) being held on a constant operation temperature above the respiration air temperature by means of separate electrical or electronical circuits maintaining the temperatures on a constant level, a measuring instrument measuring the electrical energy for compensating the cooling of one sensor respectively by inspiration or expiration as a value for the air throughput, and with calculating the difference of the energy supply values for determination of the flow direction, characterized in that the other sensor (35) has a radical interspace from the axial plane containing the first sensor (40) and the air resistance body (42) sufficient to hold it out of the area of flow influence produced by the air resistance body (42), whereby this sensor (35) serves for measuring the air throughput independently from the flow direction.

2. Spirometer according to claim 1, characterized in that the sensors (35, 40) consist of thin platinum wires respectively.

3. Spirometer according to claim 2, characterized in that the platinum wires (35, 40) are arranged between a pair of pins (31, 33 and 36, 38) respectively and extend paralelly with one another and crosswise with respect to the air flow direction.

4. Spirometer according to one of the claims 1 to 3, characterized in that the air resistance body (42) consists of a bolt or bridge extending through the air flow channel (24) crosswise with respect to tha air flow direction.

5. Spirometer according to one of the claims 1 to 4, characterized in that the air flow channel (24) is composed of a contracting portion (18), a middle portion (22) having a constant cross-section and an enlarging portion (20) and that the sensors (35, 40) and the air resistance body (42) are coordinated to the middle portion (22).

1|1

FIG.1

FIG.2

FIG.3